# EUROPÄISCHE PATENTSCHRIFT

(11) **EP 3 174 848 B1**
(45) Veröffentlichungstag und Bekanntmachung des Hinweises auf die Patenterteilung: **01.08.2018**
(21) Anmeldenummer: 15771843.8
(22) Anmeldetag: 30.07.2015
(51) Int. Cl.: A61K 45/06, C07C 277/08, A61K 31/155

(54) **VERFAHREN ZUR HERSTELLUNG VON POLYGUANIDINEN**
METHOD FOR PRODUCING POLYGUANIDINES
PROCÉDÉ DE PRÉPARATION DE POLYGUANIDINES

(30) Priorität: 31.07.2014 AT 6092014
(43) Veröffentlichungstag der Anmeldung: 07.06.2017
(73) Patentinhaber: Sealife Pharma GmbH, 3430 Tulln (AT)
(72) Erfinder: PRETSCH, Alexander, 3542 Jaidhof (AT); NAGL, Michael, 1020 Wien (AT); WIESNER, Christoph, 1160 Wien (AT); HOLLAUS, Ralph, 1170 Wien (AT); GENOV, Miroslav, 3430 Tulin (AT)
(74) Vertreter: Ellmeyer, Wolfgang
(86) Internationale Anmeldenummer: PCT/AT2015/050187
(87) Internationale Veröffentlichungsnummer: WO 2016/015081

(56) Entgegenhaltungen:
- US-A- 2 325 586
- US-A- 2 882 156
- US-A- 3 869 478
- US-A- 3 901 944
- DAFU WEI ET.AL.: "Condensation between guanidine hydrochloride and diamine/multi-amine and its influence on the structures and antibacterial activity of oligoguanidines", E-POLYMERS, Nr. 072, 26. August 2012 (2012-08-26), Seiten 1-10, XP002751231,
- R. GROSS ET.AL.: "Beschleunigung von Substitutionsreaktionen eines Phosphorsäurediesters durch Bis(guanidinium)-Verbindungen", LIEBIGS ANN. CHEM., 1994, Seiten 49-58, XP002751232,
- U. BATTAGLIA ET.AL.: "A short synthesis of triazolopyrimidine antibiotic Essramycin", J. NAT. PROD., Bd. 73, 2010, Seiten 1938-1939, XP002752052,

## Beschreibung

Die vorliegende Erfindung betrifft ein neues Verfahren zur Herstellung von Polyguanidinen, auf diese Weise hergestellte Polykondensationsprodukte und deren Verwendung als antimikrobielle oder antiinfektive Mittel.

### STAND DER TECHNIK

Polyguanidine der nachstehenden allgemeinen Formel wie auch diverse Derivate davon sind seit langem bekannt.

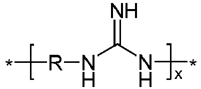

In der Patentliteratur wurden bereits 1943 im US-Patent 2.325.586 mehrere Herstellungsverfahren verschiedener Polyguanidine durch Polykondensation von i) Guanidin oder Salzen davon, ii) Cyanhalogeniden, iii) Dicyanamiden oder iv) Isocyaniddihalogeniden mit Diaminen oder v) zweier Dicyandiamide miteinander (was Cyano-substituierte Polyguanidine ergibt), sowie die Verwendung der so erzeugten Polyguanidine als Färbehilfsmittel beschrieben:
i) 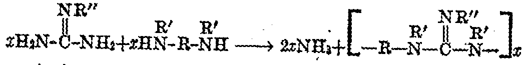
ii) 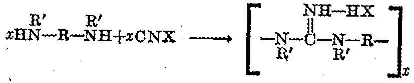
iii) 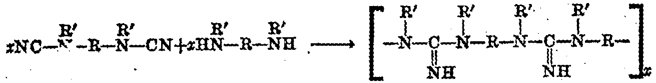
iv) 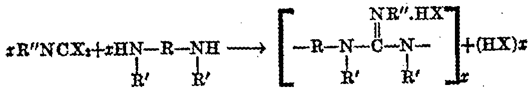
v) 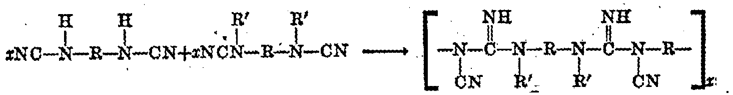
Als Diamine in den Reaktionen i) bis iv) wurden bereits damals sowohl Alkylen- und Phenylendiamine als auch Oxyalkylen- oder Polyetherdiamine, wie sie später auch als Jeffamine® bekannt werden sollten, offenbart.
Jahrzehnte später haben sich derartige Polyguanidine auch als ausgezeichnete Biozide erwiesen. So offenbart eine Gruppe um Oskar Schmidt in WO 99/54291 A1 die Herstellung von mikrobioziden Polyhexamethylenguanidinen, in WO 01/85676 A1 biozide Polyguanidine, die durch Kondensation von Guanidin mit Polyoxyalkylenen hergestellt werden, und in WO 2006/047800 A1 als Biozide, insbesondere als Fungizide, wirkende Polyguanidinderivate, die durch Polykondensation von Guanidin mit einem Gemisch aus Alkylendiamin und Oxalkylendiamin gebildet werden und geringere Toxizität aufweisen sollen als die Polymere, die nur eine der beiden Arten des zweiwertigen Rests R₁ enthalten. Dafu Wei, et. al. e-Polymers, Nr. 072, 26. August 2012, Seiten 1-10, lehrt die antibakterielle Wirkung von Guanidin Derivaten. In WO 02/30877 A1 werden ähnliche Polyguanidine als Desinfektionsmittel beschrieben, die zusätzlich Phenylengruppierungen in den Ketten enthalten. Eine russische Forschergruppe (Tets, Tets und Krasnov) offenbart in WO 2011/043690 A1, von der US 2011/0269936 A1 und EP 2.520.605 A1 abgeleitet wurden, biozide Polyguanidine der nachstehenden Formel, die durch Polykondensation von Guanidin und Hexamethylendiamin in Gegenwart von Hydrazinhydrat hergestellt werden: 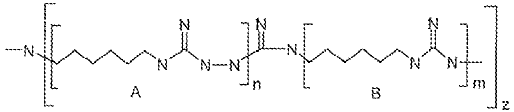 Das Hydrazin ersetzt somit während der Polykondensation - zumindest formal - eine Aminogruppe entweder nur einer oder auch zweier Guanidin-Gruppierungen, wodurch Block-Copolymere erhalten werden sollen, in denen sich Poly(hexamethylenguanidin)-Blöcke mit Poly(hexamethylenaminoguanidin)-Blöcken abwechseln und die beiden Arten von Blöcken jeweils über Guanidin-Dimere miteinander verknüpft sind, wie nachstehend dargestellt ist:

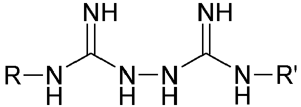

Auch diese Polymere und Säureadditionssalze davon sollen als Biozide gegen Bakterien, Viren und Pilze wirken. Allerdings finden sich in den Beispielen dieser Anmeldungen, in denen 7 verschiedene Polymere hergestellt wurden, außer der Angabe, dass jenes aus Beispiel 1 eine "feste, nahezu farblose, transparente Substanz" sei, keinerlei physikalische Daten zu den erhaltenen Produkten.

Bezüglich der möglichen Strukturen, die während der Polykondensationen von Guanidinen mit Diaminen entstehen können, existieren mehrere Artikel einer Forschergruppe der Technischen Universität Graz, z.B. Albert et al., Biomacromolecules 4(6), 1811-1817 (2003), und Feiertag et al., Macromol. Rap. Comm. 24(9), 567-570 (2003). Zusätzlich zu den verschiedenen Möglichkeiten der Terminierung der linearen Polymerketten mit jeweils einem der Ausgangsmonomere bilden sich üblicherweise in einem nicht zu vernachlässigenden Anteil, der u.a. von der Kettenlänge des Diamins abhängt, auch zyklische Moleküle der folgenden allgemeinen Formel aus:

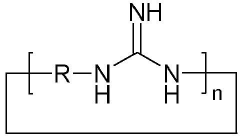

Die Hauptnachteile praktisch aller der oben beschriebenen Polyguanidin-Derivate liegen zum einen in einer nicht zu vernachlässigenden Toxizität dieser Produkte sowie - im Fall des Einsatzes hochreaktiver Komponenten - in vergleichsweise aufwändigen Herstellungsverfahren, sowie auch in der Verwendung von aus toxikologischer Sicht bekanntermaßen problematischen Komponenten wie Hydrazin, weswegen die vorliegenden Erfinder nach Lösungen zu forschen begonnen haben.

Im Zuge ihrer Forschungen hatten die Erfinder herausgefunden, dass Polykondensationsprodukte von Amino- und Diaminoguanidin mit Aminen überraschenderweise deutlich geringere Toxizität als die strukturell ähnlichen Polykondensate mit Guanidin aus den oben zitierten Dokumenten WO 2011/043690 A1, US 2011/0269936 A1 und EP 2.520.605 A1 zeigen, aber ebenfalls wirksame antimikrobielle Substanzen sind.

Diese Ergebnisse werden in den anhängigen Patentanmeldungen AT A 53/2013 und PCT/AT2014/050026 offenbart, in denen Polyguanidin-Derivate der nachstehenden Formel sowie Salze davon beansprucht werden: 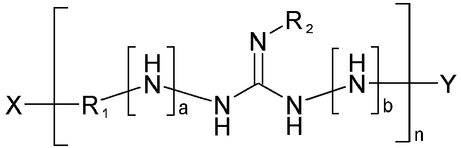 worin
X aus -NH₂, Aminoguanidino und 1,3-Diaminoguanidino ausgewählt ist;
Y aus -H und -R₁-NH₂ ausgewählt ist;
oder X und Y zusammen für eine chemische Bindung stehen, um eine zyklische Struktur zu ergeben;
R₁ aus zweiwertigen organischen Resten mit 2 bis 20 Kohlenstoffatomen ausgewählt ist, in denen gegebenenfalls ein oder mehrere Kohlenstoffatome durch O oder N ersetzt sind;
a und b jeweils 0 oder 1 sind, wobei a+b ≠ 2 ist, wenn keine 1,3-Diaminoguanidin-Einheiten enthalten sind;
R₂ aus -H und -NH₂ ausgewählt ist,
   wobei
   R₂-NH₂ ist, wenn a+b = 0 ist,
   R₂-H oder -NH₂ ist, wenn a+b = 1 ist und
   R₂-H ist, wenn a+b = 2 ist; und
n ≥ 2 ist.

Als Verfahren zur Herstellung dieser neuen Poly(di)aminoguanidine wurden - in Analogie zum damals bekannten Stand der Technik - entsprechende Diamine mit Amino- und/oder Diaminoguanidin durch Erhitzen polykondensiert.

Ohne sich auf eine Theorie festlegen zu wollen, nehmen die Erfinder an, dass Amino- und Diaminoguanidino-Gruppierungen (nachstehend kollektiv als "Aminoguanidine" bezeichnet, sofern aus dem Kontext nichts anderes hervorgeht) für humane eukaryotische Zellen besser verträglich sind als Guanidino-Gruppierungen und insbesondere als jene Polymere, die die oben dargestellten hydrazoverbrückten Guanidin-Dimere enthalten.

Allerdings haben sich auch manche dieser neuen Aminoguanidin-Verbindungen in Bezug auf antimikrobielle Wirksamkeit bzw. Toxizität nicht als vollständig zufrieden stellend erwiesen, und auch das Herstellungsverfahren ist insofern verbesserungswürdig, als es bei Verwendung bestimmter Diamine sehr hohe Temperaturen für die Schmelzpolymerisation erfordert und auch weiterhin einen mitunter problematischen Restmonomergehalt mit sich bringt.

Das Ziel der vorliegenden Erfindung war daher die Bereitstellung weiterer Polyguanidin-Derivate mit noch besseren Eigenschaften sowie eines vorteilhaften Herstellungsverfahrens dafür.

### OFFENBARUNG DER ERFINDUNG

Dieses Ziel erreicht die Erfindung in einem ersten Aspekt durch Bereitstellung eines Verfahrens zur Herstellung von Polykondensationsprodukten von Guanidin, Aminoguanidin oder Diaminoguanidin G mit einem oder mehreren Benzyl- oder Allyl-Derivaten BA nach dem folgenden Reaktionsschema: 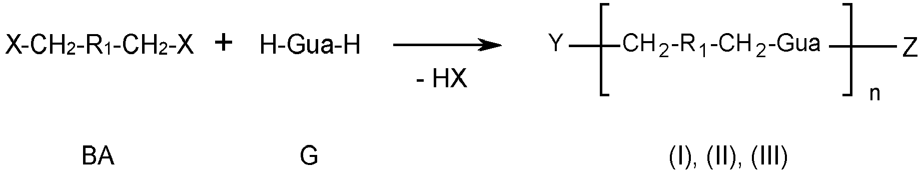 worin
X jeweils unabhängig für eine Abgangsgruppe steht;
R₁ jeweils unabhängig entweder für ein aromatisches Ringsystem mit zumindest einem aromatischen Ring, das gegebenenfalls ein oder mehrere Heteroatome, ausgewählt aus O, N und S enthält und das gegebenenfalls mit einer oder zwei Vinylgruppen substituiert ist, an die die Gruppe(n) -CH₂-X gebunden ist/sind, oder für Ethylen steht;
Gua für einen Guanidindiyl-, Aminoguanidindiyl-oderDiaminoguanidindiyl-Rest steht; Y für H-Gua steht und
Z für H steht; oder
Y und Z zusammen für eine chemische Bindung stehen, um eine zyklische Struktur zu ergeben;
wobei zumindest ein Benzyl- oder Allyl-Derivat BA einer Polykondensationsreaktion mit einem Überschuss an Guanidin, Aminoguanidin oder Diaminoguanidin G unter Abspaltung von HX unterzogen wird, um ein Polyguanidin der nachstehenden Formel (I), (II) oder (III): 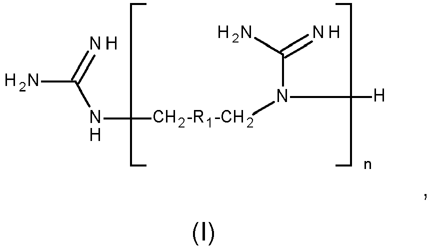 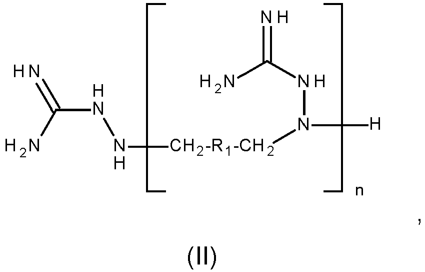 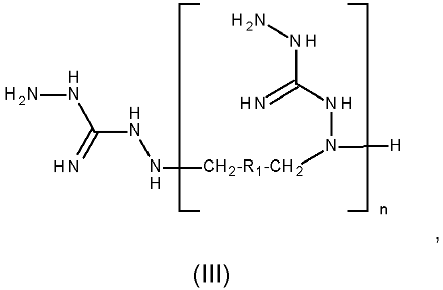 oder mit einer durch Ringschluss unter Eliminierung eines entsprechenden Guanidins erhaltenen zyklischen Struktur, oder ein Salz des Polyguanidins zu ergeben.

In diesem Herstellungsverfahren erfolgt die Polykondensation im Gegensatz zum Stand der Technik nicht unter Abspaltung von Ammoniak, sondern unter Abspaltung der Abgangsgruppe X, vorzugsweise als Halogenwasserstoff, z.B. HCl oder HBr, oder als Sulfonsäure, z.B. CH₃SO₂OH (MsOH), der/die mit den im Molekül befindlichen Amino- oder Iminogruppen Säureadditionssalze bildet, wodurch sich die Verwendung eines Säurefängers erübrigt.

Dies hat weiters zur Folge, dass die Polykondensation nicht notwendigerweise in der Schmelze durchgeführt zu werden braucht, obwohl aus Gründen der Verfahrensökonomie auch gemäß vorliegender Erfindung Schmelzpolymerisation die bevorzugte Reaktionsführung ist. Daher wird in bevorzugten Ausführungsformen das zumindest eine Benzyl- oder Allyl-Derivaten BA mit Guanidin, Aminoguanidin oder Diaminoguanidin G durch Erhitzen der Reaktanten auf eine Temperatur oberhalb ihrer Schmelztemperaturen umgesetzt, wobei die Polymerisationsreaktion vorzugsweise über einen Zeitraum von zumindest 2 h, noch bevorzugter zumindest 3 h, durchgeführt wird. Insbesondere wird die Reaktion - in Analogie zum früheren Verfahren der Erfinder - in zwei Stufen bei unterschiedlichen Temperaturen, einer ersten, niedrigeren und einer zweiten, höheren Temperatur, durchgeführt, um für möglichst vollständigen Umsatz und dadurch größere Kettenlängen bei gleichzeitig verringertem Restmonomergehalt zu sorgen.

Überraschenderweise jedoch haben die Erfinder festgestellt, dass bei Verwendung von benzylischen oder allylischen Strukturen zwar wie im Stand der Technik Gemische von Polykondensationsprodukten unterschiedlicher Stukturen entstehen. Allerdings entsprechen die Hauptprodukte nicht den aus den früheren Anmeldungen der Erfinder bekannten Strukturen mit jeweils nur einfach substituiertem Stickstoff, sondern bereits einfach substituierter Stickstoff reagiert offenbar bevorzugt ein zweites Mal, um die obigen Strukturen der Formeln (I) bis (III) zu ergeben.

Ohne sich auf eine Theorie festlegen zu wollen, nehmen die Erfinder an, dass die Reaktivität der Edukte, die auf Mesomeriestabilisierung des Übergangszustands während der nukleophilen Substitution an der benzylischen oder allylischen Methylengruppe zurückzuführen ist, zusammen mit der erhöhten Reaktivität der primär gebildeten, einfach substituierten Stickstoffaddukte zur gegebenen Stickstoff-Doppelsubstitution führt, so dass weiters anzunehmen ist, dass Ähnliches bei zumindest einem Großteil der bekannten benzylischen und allylischen Strukturen festzustellen sein wird, d.h. bei Strukturen mit einer Methylengruppe, die an einen aromatischen Ring oder an eine Doppelbindung gebunden ist, oder auch Kombinationen davon, d.h. im Falle von cinnamylischen Strukturen, bei denen - in Bezug auf Benzylreste - offenbar das bekannte Vinylogie-Prinzip zur Wirkung kommt (vgl. das spätere Beispiel 8). Letzteres ist natürlich auch auf konjugierte Doppelbindungen in aliphatischen Resten anwendbar, wie z.B. im Falle von Butadien anstelle von Ethylen. Aus diesem Grund sollen derzeit auch etwaige weitere Substituenten an diesen aromatischen Ringen und Doppelbindungen nicht speziell eingeschränkt sein, solange dadurch die Aromatizität des jeweiligen Rings nicht aufgehoben oder die Elektronendichte im aromatischen Ring oder an der Doppelbindung erheblich verändert wird, speziell im Falle von Tautomerieeffekten, wie z.B. Keto-Enol, Imin-Enamin usw.

In den gegebenen Strukturen der Formeln (I) bis (III) sind die Guanidin- bzw. Aminoguanidin-Einheiten über das doppelt in die Kette eingebundene Stickstoffatom, außerhalb der Kette positioniert, wobei der Strukturtyp der Formel (I), (II) bzw. (III) in Übereinstimmung mit der spektroskopischen Evidenz in der Mehrzahl der gebildeten Oligomerspezies vorliegt.

Der erhaltene Verknüpfungstyp der neuen Polyguanidine wurde mittels HMBC-NMR ermittelt: So sind beispielsweise beim Polyaminoguanidin aus Beispiel 1 die entsprechenden Long-Range-Kopplungen der über einen Stickstoff verbundenen benzylischen CH₂-Protonen (AB-System bei 3,8 und 4,2 ppm) über dieses jeweils in die Oligomer-Kette eingebundene N-Atom sowie die beiden benzylischen Kohlenstoffatome (bei 64 ppm) nachweisbar. Als Hinweis auf höher verzweigte Nebenkomponenten (∼15 % laut ¹H-NMR) wurden des weiteren Signale, die mit einer Benzylierung eines weiteren, jenseits der Iminofunktionalität liegenden Guanidinstickstoffs korellieren, vorgefunden (AB-System bei 4,3 und 4,5 ppm, HMBC-Long-Range-Signale in die Guanidinkohlenstoffregion bei 160 ppm). Eine weitere Gruppe von NMR-Signalen (¹H-Shift bei 8 ppm, ¹³C-Shift bei 150 ppm) benzylischer Iminofunktionalitäten sind korrelliert mit Oligomer-Pendents vom Sommelet-Oxidationstypus, was in Übereinstimmung mit den massenspektroskopischen Daten (Doppellinien vom Typ m/z [M-2] für alle Oligomere) steht..

Von diesem neuen Strukturtyp wurde von den Erfindern sogar noch bessere antimikrobielle Aktivität erhofft als von ihren früheren Polyaminoguanidinen, was auch bestätigt werden konnte, wie die nachstehenden Ausführungsbeispiele der Erfindung belegen: Die Biozid-Aktivität ist deutlich gesteigert, während gleichzeitig die Toxizität noch etwas geringer ausfällt.

Letzteres, nehmen die Erfinder an, ohne sich auf eine Theorie festlegen zu wollen, dürfte unter anderem auf die gegenüber den früheren Polyaminoguanidinen höheren mittleren Kettenlängen sowie den noch niedrigeren Restmonomergehalt zurückzuführen sein.

Zur Optimierung der Reaktionsbedingungen, um einen möglichst guten Kompromiss zwischen Reaktionszeit, Kettenlänge und Restmonomergehalt zu finden, haben die Erfinder Versuchsreihen mit unterschiedlichen Verhältnissen zwischen dem Benzyl- oder Allyl-Derivat BA und den Guanidinen G, verschiedenen Temperaturen sowie unterschiedlichen Reaktionszeiten durchgeführt und herausgefunden, dass bei einem Verhältnis G/BA knapp unter 2 die so erhaltenen Produkte die besten biologischen Ergebnisse geliefert haben, wobei die Reaktionsgemische vorzugsweise zunächst 2 bis 3 h lang auf eine Temperatur von etwa 150-170 °C und anschließend 1 bis 2 h lang auf eine Temperatur von 180-190 °C erhitzt werden sollten.

In einem zweiten Aspekt der Erfindung werden durch die vorliegende Erfindung neue Polyguanidine bereitgestellt, die den nachstehenden Formeln (I) bis (III) entsprechen, nämlich ein Polyguanidin, das der nachstehenden Formel (I) entspricht: 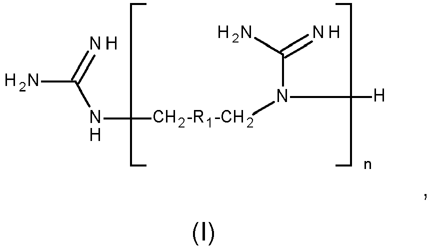 oder das eine durch Ringschluss unter Eliminierung eines Guanidins erhaltene zyklische Struktur aufweist;
ein Polyguanidin, das der nachstehenden Formel (II) entspricht: 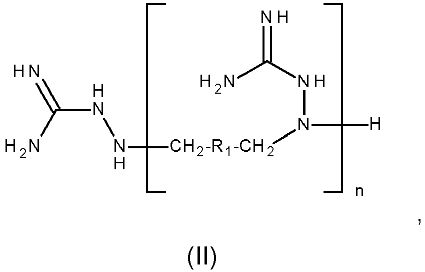 oder das eine durch Ringschluss unter Eliminierung eines Aminoguanidins erhaltene zyklische Struktur aufweist, sowie
ein Polyguanidin, das der nachstehenden Formel (III) entspricht: 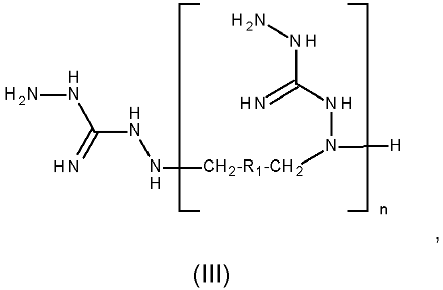 oder das eine durch Ringschluss unter Eliminierung eines Diaminoguanidins erhaltene zyklische Struktur aufweist;
worin R₁ entweder für ein aromatisches Ringsystem mit zumindest einem aromatischen Ring, das gegebenenfalls ein oder mehrere Heteroatome, ausgewählt aus O, N und S enthält und das gegebenenfalls mit einer oder zwei Vinylgruppen substituiert ist, an die die Gruppe(n) -CH₂-X gebunden ist/sind, oder für Ethylen steht und in bevorzugten Ausführungsformen aus zweiwertigen Resten von gegebenenfalls substituiertem Benzol, Divinylbenzol, Furan, Pyrrol, Thiophen, Pyridin, Biphenyl, Fluoren und Ethylen ausgewählt ist, noch bevorzugter aus aus zweiwertigen Resten von Benzol, Divinylbenzol, Pyridin, Biphenyl und Ethylen, die bereits gute Ergebnisse geliefert haben.

Aufgrund der hohen antimikrobiellen Wirksamkeit der neuen Strukturen stellt die Erfindung in einem dritten Aspekt ein oben definiertes neues Polyguanidin zur Verwendung als Antibiotikum und Antiinfektivum bereit, vorzugsweise zur Bekämpfung von bakteriellen, viralen und Pilz-Infektionen bei einem menschlichen oder tierischen Patienten. Das Polyguanidin kann dabei zur topischen oder systemischen Verabreichung dienen, vorzugsweise zur Verabreichung in Form eines Arzneimittels oder einer pharmazeutischen Zusammensetzung.

Alternativ dazu können die neuen Polyguanidine aber auch als antimikrobielle Mittel *ex vivo* eingesetzt werden, vorzugsweise als Wirkkomponente von antimikrobiellen Anstrichen, Beschichtungen, Folien oder Membranen oder dergleichen.

In einem vierten Aspekt stellt die Erfindung demnach ein Arzneimittel oder eine pharmazeutische Zusammensetzung zur Bekämpfung von bakteriellen, viralen und Pilz-Infektionen bei einem menschlichen oder tierischen Patienten bereit, das/die zumindest eines der neuen Polyguanidine als Antiinfektivum umfasst und vorzugsweise weiters zumindest einen pharmazeutisch annehmbaren Träger oder Exzipienten und gegebenenfalls ein oder mehrere Adjuvantien und/oder einen oder mehrere andere Wirkstoffe umfasst.

Bevorzugt bevorzugt umfasst das Arzneimittel oder die pharmazeutische Zusammensetzung zumindest einen anderen Wirkstoff, der ebenfalls antimikrobiell wirkt, um die Wirkung zu verstärken und etwaige Synergieeffekte zu nutzen. Der zumindest eine andere Wirkstoff kann dabei mitunter auch gegen ein anderes Leiden als eine bakterielle Infektion wirksam sein. Nur als Beispiele seien Antidiarrhoika und so genannte Magenschoner erwähnt.

Nachstehend wird die Erfindung anhand von nichteinschränkenden Beispielen näher beschrieben.

### BEISPIELE

### Beispiel 1

### Herstellung von Polyaminoguanidin (1)

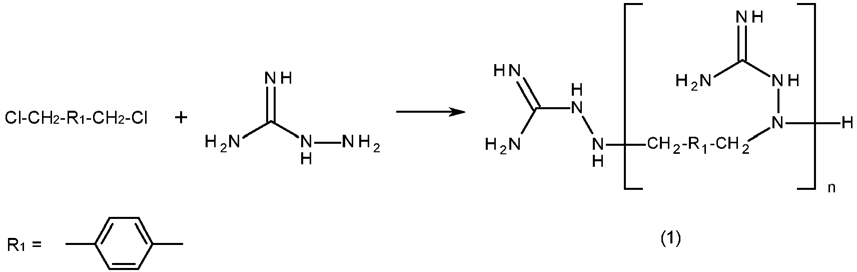

α,α'-Dichlor-p-xylol (880 mg, 5,03 mmol) und 1,95 Äquivalente an Aminoguanidin-hydrochlorid (1083 mg, 9,80 mmol) wurden in einem offenen Reaktionsgefäß unter Rühren zunächst 3 h lang auf 160 °C, danach 2 h lang auf 180 °C erhitzt. Nach Abkühlen des Reaktionsgemischs auf unter 80 °C wurde zum Reaktionsprodukt etwa die zehnfache Menge an Wasser zugesetzt, und nach gründlichem Durchmischen mittels Rühren oder Ultraschallbehandlung wurde eine klare, leicht gelbliche Lösung mit Spuren fester Anteile erhalten. Diese wurde durch eine 0,2-µm-PFTE-Membran filtriert und danach eingedampft, um Polyguanidin (1) als gelblichen, amorphen Feststoff zu erhalten.

Zur Analyse wurde eine Probe in der zehnfachen Menge an D₂O gelöst. Bei der Aufnahme des ¹H- und des ¹³C-NMR-Spektrums wurde zur Referenzierung DSS (4,4-Dimethyl-4-silapentan-1-sulfonsäure) als interner Standard zugesetzt:
¹H-NMR (D₂O), δ (ppm): 3,72-3,91 (ad, CH_{2A}-N(Gua)-CH_{2A}, *J*_{A,B} = 12,4 Hz, CH_{2A} Kette), 3,93-4,05 (as, CH₂-NH-Gua, CH₂ terminal), 4,10-4,23 (ad, CH_{2B}-N(Gua)-CH_{2B}, *J*_{A,B} = 12,4 Hz, CH_{2B} Kette), 4,29-4,39 (m, CH_{2A} α-Gua), 4,45-4,52 (m, CH_{2B} α-Gua), 7,30-7,83 (m, =CH Ar), 8,08 (as, N=CH).
¹³C-NMR (D₂O), δ (ppm): 46,25, 46,56, 46,94 (CH₂ α-Gua), 56,90, 56,97, 57,03 (CH₂ terminal), 63,87, 64,02 (CH₂-N(Gua)-CH₂ Kette), 128,93, 129,04, 129,57, 129,63, 129,78, 129,84, 130,20, 130,32, 130,49, 130,66, 132,10, 132,17, 132,30, 132,40, 132,62, 132,67, 132, 75, 132,83, 132,92, 133,20 (CH Ar), 135,02, 135,19, 137,54, 137,92, 138,13, 138,50, 139,07, 139,23, 141,31, 142,53 (C_{q} Ar), 150,21, 151,05, 151,12 (N=CH), 157,60, 159,67, 159,73, 160,85 (C_{q} Gua).

Die NMR-Signale im Bereich 3,72-3,91 ppm und 4,10-4,23 ppm (¹H-Achse) und bei 64,02 ppm (¹³C-Achse) bestätigen das Vorliegen eines zweifach substituierten Stickstoffatoms des Aminoguanidins.
MALDI-MS - MALDI-TOF (im Positivionenmodus (Matrixunterdrückung aus)); Scan 20-3000 m/z (Deflexion aus); Matrix: ACH (a-Cyano-4-hydroxyzimtsäure); (m/z): 247,3, 249,3, 251,4, 425,3, 427,3, 601,4, 603,4, 777,5, 779,5, 953,7, 955,7, 1129,8, 1131,9, 1306,0, 1308,0, 1482,1, 1484,1, 1658,0, 1660,0, 1834,1, 1836,1, 2010,2, 2012,2, 2186,3, 2188,3, 2362,4.

### Beispiel 2

### Herstellung von Polyaminoguanidin (2)

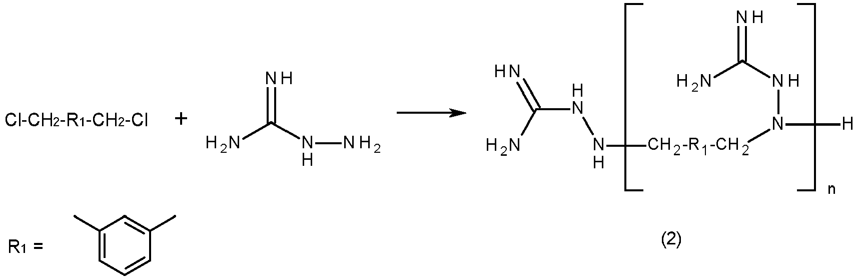

Auf analoge Weise wie in Beispiel 1 wurde aus α,α'-Dichlor-m-xylol und Aminoguanidin-hydrochlorid Polyguanidin (2) als gelblicher, amorpher, vollständig in Wasser löslicher Feststoff erhalten.
¹H-NMR (D₂O), δ (ppm): 3,73-3,92 (ad, CH_{2A}-N(Gua)-CH_{2A}, *J*_{A,B} = 12,7 Hz, CH_{2A} Kette), 3,94-4,05 (as, CH₂-NH-Gua, CH₂ terminal), 4,10-4,23 (ad, CH_{2B}-N(Gua)-CH_{2B}, *J*_{A,B} = 12,7 Hz, CH_{2B} Kette), 4,29-4,38 (m, CH_{2A} α-Gua), 4,45-4,53 (m, CH_{2B} α-Gua), 7,23-7,85 (m, =CH Ar), 8,10 (as, N=CH).
¹³C-NMR (D₂O), δ (ppm): 46,36, 46,66, 47,01 (CH₂ α-Gua), 57,01, 57,04, 57,12, 57,14 (CH₂ terminal), 63,94 (CH₂-N(Gua)-CH₂ Kette), 129,63, 129,75, 130,09, 130,20, 130,83, 131,38, 131,44, 131,53, 131,57, 131,67, 131,82, 131,89, 132,18, 132,34, 132,73, 133,52, 134,23, 134,52, 135,29 (CH Ar), 135,72, 135,81, 136,12, 138,59, 138,69, 138,73, 139,13, 139,77, 139,90, 140,30 (C_{q} Ar), 151,24 (N=CH), 157,67, 159,78, 159,81, 160,86 (C_{q} Gua).

Die NMR-Signale im Bereich 3,73-3,92 ppm und 4,10-4,23 ppm (¹H-Achse) und bei 63,94 ppm (¹³C-Achse) bestätigen wiederum das Vorliegen eines zweifach substituierten Stickstoffatoms des Aminoguanidins.
MALDI-MS - MALDI-TOF (m/z): 247,3, 249,3, 251,4, 425,3, 427,3, 601,4, 603,4, 777,5, 779,5, 953,7, 955,7, 1129,8, 1131,9, 1306,0, 1308,0, 1482,1, 1484,1, 1658,0, 1660,0, 1834,1, 1836,1, 2010,2, 2012,2, 2186,3, 2188,3.

### Beispiel 3

### Herstellung von Polyaminoguanidin (3)

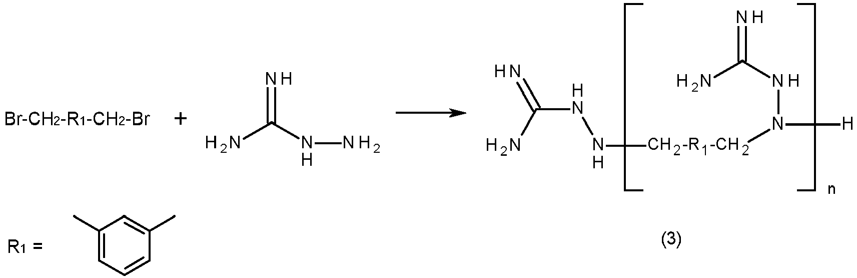

Auf analoge Weise wie in Beispiel 2 wurde aus 132 mg (0,5 mmol) α,α'-Dibrom-m-xylol (anstelle des Dichlor-Derivats) sowie 1,75 Äquivalenten an Aminoguanidin-hydrochlorid (97 mg, 0,88 mmol) Polyguanidin (3) als bräunlicher, amorpher, wasserlöslicher Feststoff erhalten.
¹H-NMR (D₂O), δ (ppm): 3,63-3,95 (m, CH_{2A}-N(Gua)-CH_{2A}, CH_{2A} Kette), 3,95-4,08 (as, CH₂-NH-Gua, CH₂ terminal), 4,13-4,24 (ad, CH_{2B}-N(Gua)-CH_{2B}, *J*_{A,B} = 12,5 Hz, CH_{2B} Kette), 4,31-4,40 (m, CH_{2A} α-Gua), 4,47-4,55 (m, CH_{2B} α-Gua), 7,17-7,86 (m, =CH Ar), 8,12 (as, N=CH).
¹³C-NMR (D₂O), δ (ppm): 46,38, 46,64, 46,99 (CH₂ α-Gua), 56,98, 57,11,57,48 (CH₂ terminal), 63,90 (CH₂-N(Gua)-CH₂ Kette), 128,58, 129,08, 129,64, 129,76, 130,05, 130,20, 130,81, 130,98, 131,35, 131,41, 131,51, 131,71, 131, 80, 131,87, 132,16, 132,33, 132,69, 133,49, 134,21, 134,51, 135,29 (CH Ar), 135,66, 135,76, 136,06, 138,68, 138,98, 139,07, 139,25, 139,72, 139,85, 140,25 (C_{q} Ar), 150,46, 151,29 (N=CH), 159,73, 160,84 (C_{q} Gua).

Die NMR-Signale im Bereich 3,63-3,95 ppm und 4,13-4,24 ppm (¹H-Achse) und bei 63,90 ppm (¹³C-Achse) bestätigen wiederum das Vorliegen eines zweifach substituierten Stickstoffatoms des Aminoguanidins.
MALDI-MS - MALDI-TOF (m/z): 247,3, 249,3, 251,4, 425,3, 427,3, 601,4, 603,4, 777,5, 779,5, 953,7, 955,7, 1129,8, 1131,9, 1306,0, 1308,0, 1482,1, 1484,1, 1658,0, 1660,0, 1834,1, 1836,1, 2010,2, 2012,2, 2186,3, 2188,3.

### Beispiel 4

### Herstellung von Polydiaminoguanidin (4)

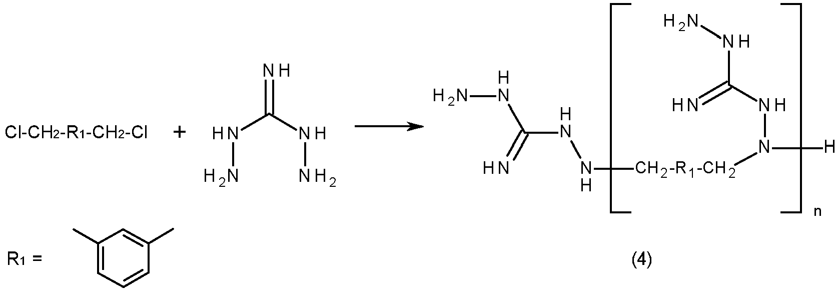

Auf analoge Weise wie in Beispiel 2 wurde aus 88 mg (0,5 mmol) α,α'-Dichlor-m-xylol und 1 Äquivalent an Diaminoguanidin-hydrochlorid (68 mg, 0,5 mmol) Polyguanidin (4) als gelblicher, amorpher, wasserlöslicher Feststoff erhalten.

Die Strukturaufklärung mittels ¹H- und ¹³C-NMR zeigte über die Anwesenheit der in den Beispielen 1 bis 3 gefundenen Produktspezies hinaus auch die Gegenwart größerer Anteile von höher verzweigten Komponenten, bei denen ein weiterer Guanidin-Stickstoff jenseits der Imino-Funktionalität benzyliert ist.

### Beispiel 5

### Herstellung von Polyguanidin (5)

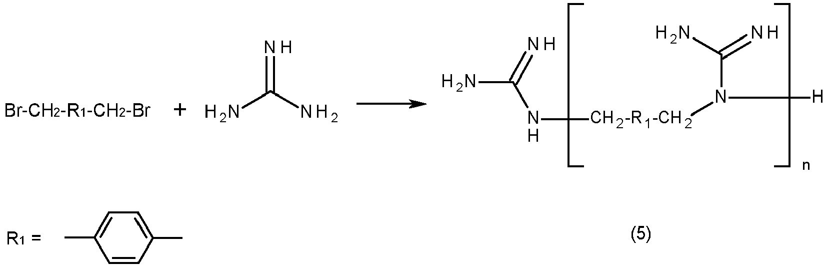

Auf analoge Weise wie in Beispiel 3 wurde aus 132 mg (0,5 mmol) α,α'-Dibrom-p-xylol und 1,75 Äquivalenten an Guanidin-hydrochlorid (83 mg, 0,88 mmol) Polyguanidin (5) als wasserlöslicher, rötlicher, amorpher Feststoff erhalten.

Die Strukturaufklärung mittels ¹H- und ¹³C-NMR zeigte über die Anwesenheit der in den Beispielen 1 bis 3 gefundenen Produktspezies hinaus auch die Gegenwart größerer Anteile von höher verzweigten Komponenten, bei denen ein weiterer Guanidin-Stickstoff jenseits der Imino-Funktionalität benzyliert ist.
MALDI-MS - MALDI-TOF (m/z): 355,3, 382,3, 516,4, 543,4, 677,3, 704,5, 838,5, 865,5, 999,6, 1026,6, 1160,7, 1187,7, 1321,8, 1348,8, 1483,9, 1510,9, 1672,0, 1833,1, 1995,2.

### Beispiel 6

### Herstellung von Polyaminoguanidin (6)

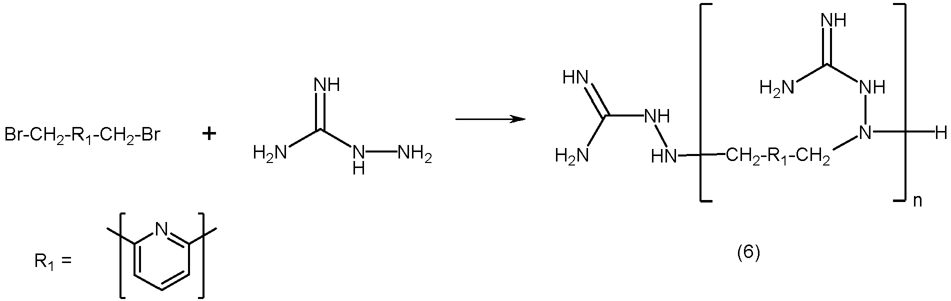

2,6-Bis(brommethyl)pyridin (265 mg, 1 mmol) und 1,95 Äquivalente an Aminoguanidin-hydrochlorid (216 mg, 1,95 mmol) wurden in einem offenen Reaktionsgefäß unter Rühren zunächst 1,5 h lang auf 160 °C, danach 1,5 h lang auf 180 °C erhitzt. Nach Abkühlen des Reaktionsgemischs auf unter 80 °C wurde zum Reaktionsprodukt Wasser (4,81 ml) zugesetzt, und nach gründlichem Durchmischen mittels Rühren oder Ultraschallbehandlung sowie Filtration durch eine 0,2-µm-PFTE-Membran wurde eine klare, dunkelbraune Lösung erhalten.
MALDI-MS - MALDI-TOF (m/z): 248,4, 250,4, 252,4, 421,4, 423,4, 425,4, 427,4, 429,4, 598,4, 600,4, 602,4, 604,4.

### Beispiel 7

### Herstellung von Polyaminoguanidin (7)

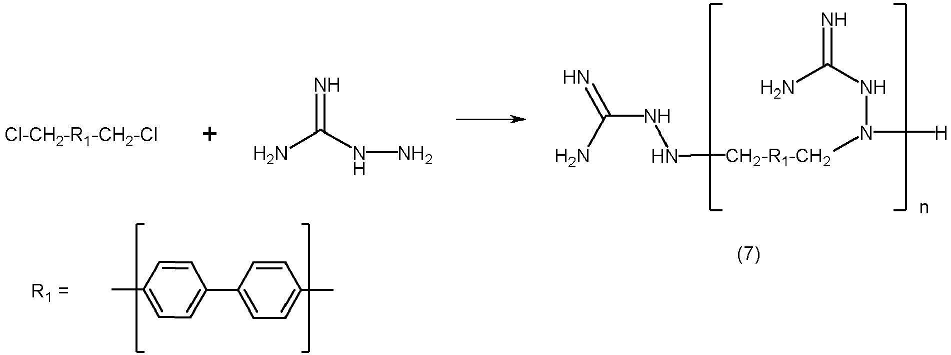

Auf analoge Weise wie in Beispiel 2 wurde aus 4,4'-Bis(chlormethyl)biphenyl (251 mg, 1 mmol) sowie 1,95 Äquivalenten an Aminoguanidin-hydrochlorid (216 mg, 1,95 mmol) Polyguanidin (7) als gelblicher, amorpher Feststoff erhalten, der abgesehen von geringen Mengen eines festen Rückstands leicht in Wasser löslich ist..
MALDI-MS - MALDI-TOF (m/z): 323,4, 325,4, 327,4, 575,4, 577,4, 579,4, 827,6, 829,6, 831,6.

### Beispiel 8

### Herstellung von Polyaminoguanidin (8)

### 8.1 Herstellung von Dimethyl-3,3'-(1,3-phenylen)-(2E,2'E)-diacrylat

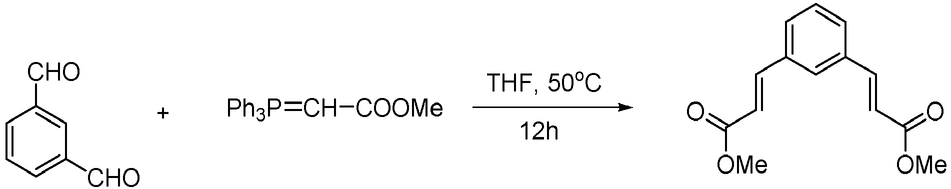

Zu einer Lösung von 0,75 mmol Isophthalaldehyd in 10 ml THF wurde unter Luftausschluss eine Lösung von 2,05 Äquivalenten (Methoxycarbonylmethylen)triphenylphosphoran (1,54 mmol) in 15 ml THF zugesetzt. Das Reaktionsgemisch wurde 12 h lang bei 50 °C gerührt und eingeengt. Chromatographische Reinigung (Kieselgel, Dichlormethan) ergab 0,62 mmol (83 % d.Th.) eines weißen Feststoffs.
¹H-NMR (400 MHz, CDCl₃): 3,82 (s, 6H), 6,47 (d, *J* = 16 Hz, 2H), 7,42 (dd, *J* = 7,7 + 7,7 Hz, 1H), 7,54 (dd, *J* = 7,7 + 1,7 Hz, 2H), 7,64 (t, *J* = 1,7 Hz, 1H), 7,69 (d, *J* = 16 Hz, 2H).

### 8.2 Herstellung von (2E,2'E)-3,3'-(1,3-Phenylen)-bis(prop-2-en-1-ol)

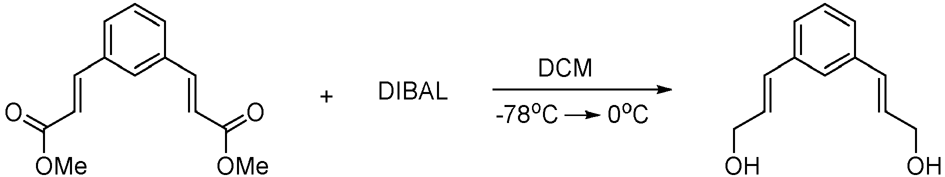

In einem Schlenkgefäß wurden 1,50 mmol Dimethyl-3,3'-(1,3-phenylen)-(2E,2'E)-diacrylat in 30 ml wasserfreiem Dichlormethan gelöst. Bei -78 °C wurden 4,5 Äquivalente Diisobutylaluminiumhydrid als 1 M Lösung in Toluol (6,75 ml) langsam zugetropft. Das Reaktionsgemisch wurde 2 h lang bei -78 °C gerührt und anschließend bei 0 °C mit Methanol hydrolysiert. Der entstandene weiße Niederschlag wurde abfiltriert, das Filtrat eingeengt und chromatographisch gereinigt (Kieselgel, DCM:EE 1:1), wobei 1,05 mmol (70 % d.Th.) eines weißen Feststoffs isoliert wurden.
¹H-NMR (400 MHz, CDCl₃) δ (ppm): 4,26 (m, 4H), 6,33 (dm, *J* = 16 Hz, 2H), 6,56 (br,d, *J* = 16 Hz, 2H), 7,22 (m, 3H), 7,34 (br s, 1H).

### 8.3 Herstellung von 1,3-Bis((E)-3-chlorprop-1-en-1-yl)benzol

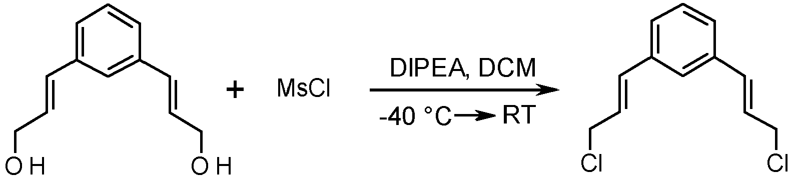

In einem Schlenkgefäß wurden 0,95 mmol Dimethyl-3,3'-(1,3-phenylen)-(2E,2'E)-diacrylat und 3 Äquivalente Diisopropylethylamin (DIPEA, 2,85 mmol) in 20 ml Dichlormethan vorgelegt und auf -40 °C abgekühlt, wonach 2,38 mmol Methansulfonylchlorid zugesetzt wurden und das Reaktionsgemisch bei Raumtemperatur 12 h lang gerührt wurde. Nach Abziehen des Lösungsmittels wurde das Rohprodukt chromatographisch gereinigt (Kieselgel, DCM), wobei 0,57 mmol (60 % d.Th.) eines weißen, kristallinen Feststoffs isoliert wurden.
¹H-NMR (400 MHz, CDCl₃) δ (ppm): 4,25 (dd, *J* = 7,1 + 1,2 Hz, 4H), 6,34 (dt, *J* = 15,7 + 7,1 Hz, 2H), 6,65 (dt, *J* = 15,7 + 1,2 Hz, 2 H), 7,30 (m, 3H), 7,40 (m, 1H).

### 8.4 Herstellung von Polyaminoguanidin (8)

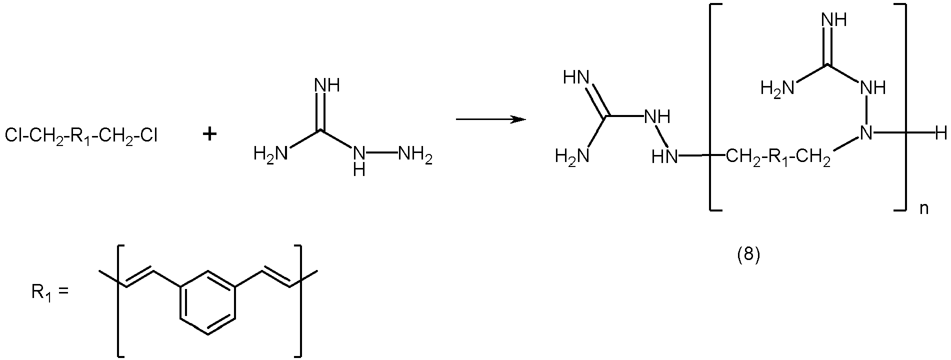

Auf analoge Weise wie in Beispiel 2 wurde aus 1,3-Bis((E)-2-chlorvinyl)benzol (200 mg, 1 mmol) und 1,95 Äquivalenten an Aminoguandin-hydrochlorid (216 mg, 1,95 mmol) Polyguanidin (8) als gelbliches, durchscheinendes, wasserlösliches Gel erhalten.
MALDI-MS - MALDI-TOF (m/z): 303,3, 531,4, 759,6, 833,7, 987,8, 1061,9, 1216,0.

### Beispiel 9

### Herstellung von Polyaminoguanidin (9)

### 9.1 Herstellung von cis-1,4-Bis(methylsulfonyloxy)but-2-en

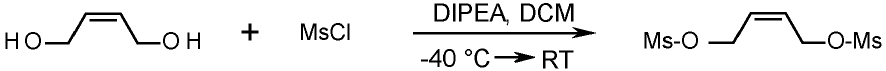

10 g cis-But-2-en-1,4-diol (113 mmol) und 3,0 Äquivalente Diisopropylethylamin (44 g, 340 mmol, 60 ml) wurden in 250 ml Dichlormethan gelöst und unter Argon-Atmosphäre auf -40 °C abgekühlt, wonach 2,4 Äquivalente Methansulfonylchlorid (30,9 g, 270 mmol, 20,9 ml) portionsweise zugegeben wurden und das Reaktionsgemisch innerhalb 1 h auf +10 °C erwärmen lassen wurde. Die klare, gelbe Lösung wurde auf 500 ml eiskaltes Wasser gegossen und die organische Phase mit weiteren 500 ml kaltem Wasser, danach mit 200 ml 2 N HCl, danach zweimal mit je 200 ml gesättigter NaHCO₃-Lösung und abschließend weitere zweimal mit je 200 ml Wasser gewaschen. Die Dichlormethan-Lösung des Produkts wurde über Na₂SO₄ getrocknet, und das Lösungsmittel wurde im Vakuum abgezogen, bis ein weißes Präzipitat auftrat, wonach die minimale Menge an Dichlormethan zugegeben wurde, um erneut eine klare Lösung zu erhalten. Nach Zugabe von 25 ml Diethylether wurde das Produkt bei -20 °C aus der Lösung auskristallisieren lassen, wonach 10 g cis-1,4-Bis(methyl-sulfonyloxy)-but-2-en als kristalliner, weißer Feststoff isoliert wurden.
¹H-NMR (400 MHz, CDCl₃) δ (ppm): 3,04 (s, 3H), 4,84 (m, 2H), 5,95 (m, 1H).

### 9.2 Herstellung von Polyaminoguanidin (9)

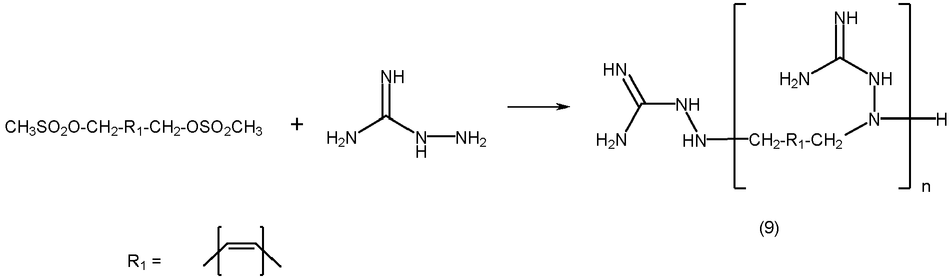

cis-1,4-Bis(methylsulfonyloxy)but-2-en (246 mg, 1 mmol) und 1,95 Äquivalente an Aminoguanidin-hydrochlorid (216 mg, 1,95 mmol) wurden in einem geschlossenen Reaktionsgefäß unter Argon-Atmosphäre unter Rühren zunächst 3 h lang auf 160 °C, danach 2 h lang auf 180 °C erhitzt. Nach Abkühlen des Reaktionsgemischs auf unter 80 °C wurde zum Reaktionsprodukt Wasser (4,67 ml) zugesetzt und so eine klare, gelbrote Lösung erhalten.
MALDI-MS - MALDI-TOF (m/z): 201,3, 251,3, 253,3, 297,2, 325,3, 327,3, 349,2, 377,3, 423,3, 451,3, 453,3, 519,3.

### Beispiel 10

### Herstellung von Polyaminoguanidin (10)

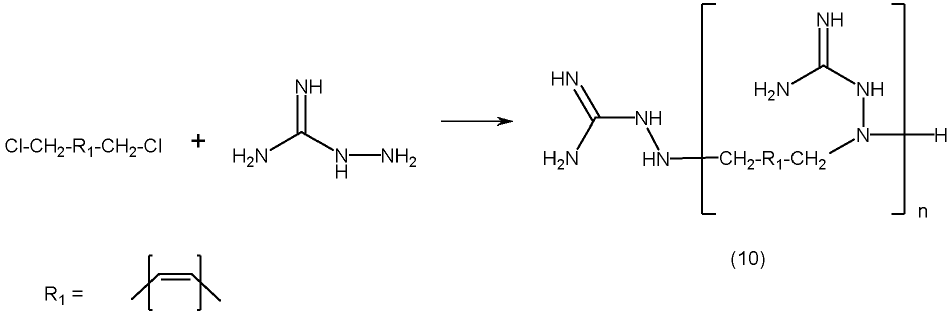

1,4-Dichlor-2-buten (262 mg, 1,3 mmol) und 1,95 Äquivalente an Aminoguandin-hydrochlorid (216 mg, 1,95 mmol) wurden in einem geschlossenen Reaktionsgefäß unter Argon-Atmosphäre unter Rühren und unter wiederholtem (dreimal pro Stunde) Austausch der Atmosphäre gegen frisches Argon zunächst 2 h lang auf 150 °C, danach 1 h lang auf 170 °C erhitzt. Nach Abkühlen des Reaktionsgemischs auf unter 80 °C wurde zum Reaktionsprodukt Wasser (4,67 ml) zugesetzt und so eine klare, gelbrote Lösung erhalten.
MALDI-MS - MALDI-TOF (m/z): 201,3, 251,3, 253,3, 297,2, 325,3, 327,3, 377,3, 423,3, 451,3, 453,3.

### Vergleichsbeispiel 1

### Herstellung eines Polyaminoguanidins aus Diamin und Aminoguanidin

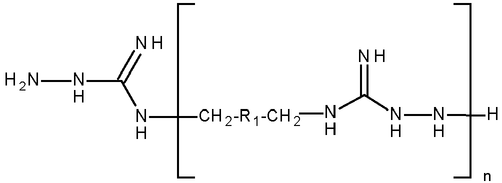

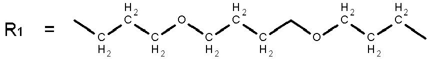

23 mmol 1,3-Diaminoguanidinium-hydrochlorid und 24 mmol 4,9-Dioxadodecan-1,12-diamin wurden in einem mit einem Trockenrohr verschlossenen Reaktionsgefäß 90 min lang unter Rühren auf 120 °C erhitzt, danach wurde die Temperatur für 100 min auf 180 °C erhöht, davon am Ende dieser Reaktionszeit für 45 min unter vermindertem Druck (50 mbar). Nach Abkühlen der Reaktionsmischung auf unter 80 °C wurden zum gelartigen Reaktionsprodukt 25 ml Wasser zugesetzt. Nach einigen Stunden wurde eine klare Lösung erhalten.

Von einer Probe der erhaltenen wässrigen Lösung wurde das Wasser abgedampft, und der erhaltene Rückstand wurde im Vakuum getrocknet, wobei eine rötliche, viskose Flüssigkeit erhalten wurde. Diese wurde in 2 ml D₂O (mit einem Deuterierungsgrad > 99,5%) gelöst, und ein ¹H-Kernresonanz-(¹H-NMR-) Spektrum wurde aufgenommen. Die Lage der auf diese Weise unterscheidbaren Gruppen von Methylen-Protonen der Reste R₁ im Produkt ist wie folgt:
¹H-NMR (D₂O), δ (ppm): 1,54-1,67 (m, OCH₂*CH₂CH₂*CH₂O), 1,80-1,95 (m, NCH₂*CH₂*), 3,23-3,38 ppm (m, N*CH₂*), 3,42-3,65 ppm (m, CH₂*CH₂*O*CH₂*CH₂).

Dies bestätigt die Struktur der verwendeten Diamin-Komponente, 4,9-Dioxadodecan-1,12-diamin.

### Beispiel 11

### Aktivitätsbestimmung: antimikrobielle/antifungale/antivirale Wirkung

Die Aktivität der neuen Verbindungen wurde in mehrfach durchgeführten Screeningsystemen getestet. Die antibakterielle und antifungale Aktivität wurde mittels MHK-Test untersucht. MHK steht für die "minimale Hemm-Konzentration" (engl.: MIC für "minimal inhibitory concentration") und bezeichnet die niedrigste Konzentration einer Substanz, bei der mit bloßem Auge keine Vermehrung von Mikroorganismen wahrgenommen werden kann. Bestimmt wird die MHK mit einem so genannten Titerverfahren, bei dem die Substanz ausverdünnt und anschließend der Erreger zugefügt wird.

In der Regel wird so die Konzentration eines Antibiotikums bestimmt, die das Wachstum eines Bakterienstammes gerade noch hemmt. Die MHK wird in Mikrogramm pro Milliliter (µg/ml) oder in Vol.-% angegeben, und die Verdünnungen erfolgen in der Regel in log2-Schritten. Hierin wurde eine Ausgangskonzentration von 1 % jeweils auf das Doppelte verdünnt, was folglich Testkonzentrationen von 0,5 %, 0,25 %, 0,125 % usw. ergab. Niedrigere Werte spiegeln demnach bessere Aktivität als Antiinfektivum wider.

Die Tests wurden nach den vom EUCAST (European Committee for Antimicrobial Susceptibility Testing) geforderten Standards und gemäß den AFST- ("Antifungal Susceptibility Testing") Vorschriften der European Society of Clinical Microbiology and Infectious Diseases (ESCMID) durchgeführt.

Das Screeningsystem für Viren ist ein Infektionssystem, bei dem Wirtszellen *in vitro* infiziert werden und die Testsubstanz vor oder nach der Infektion zugesetzt und ihre Aktivität bestimmt wird. Alle diese Tests wurden gemäß den betriebsinternen Standardvorschriften von SeaLife Pharma zum Arzneimittelscreening durchgeführt, wobei analoge Verdünnungsreihen wie im antibakteriellen/antifungalen Test eingesetzt wurden.

In der umseitigen Tabellen 1 bis 3 sind die Testergebnisse bezüglich der antiinfektiven Wirkung der erfindungsgemäßen neuen Verbindungen aus den Beispielen 1, 3, 4 und 5 und von Vergleichsbeispiel 1 gegen einige multiresistente Bakterien und Pilze sowie Viren angegeben. Die Daten sind jeweils Mittelwerte von Mehrfachbestimmungen.

Es ist klar ersichtlich, dass die neuen Verbindungen der Erfindung ausgezeichnete Aktivität sowohl gegen gram-positive als auch gram-negative Erreger zeigen.

### Beispiel 12

### Toxizitätstests

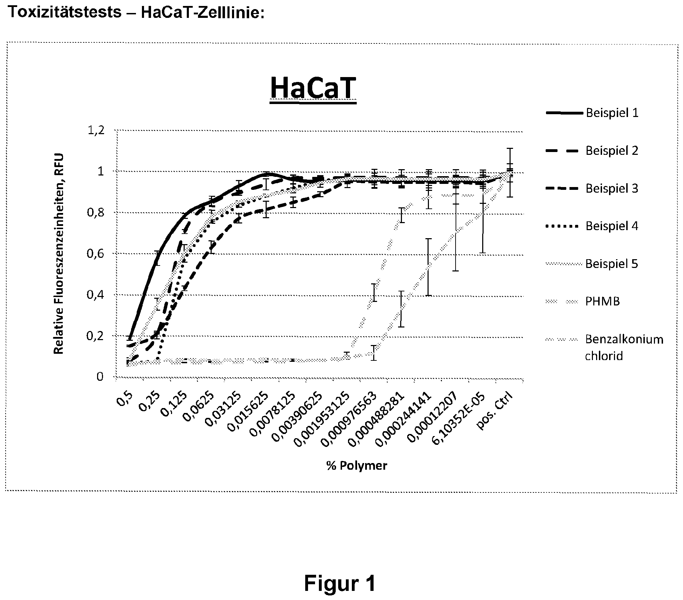

Der beiliegenden Fig. 1 ist weiters zu entnehmen, dass die erfindungsgemäßen neuen Polyguanidine in jenen Konzentrationen, bei denen sie ausgezeichnete antimikrobielle Aktivität besitzen, gleichzeitig äußerst geringe Toxizität zeigen, wie dies aus dem Anteil an überlebenden Zellen der exponierten HaCaT-Zelllinie als Zellmodell auf der Y-Achse klar hervorgeht.

**Tabelle 1 - Wirkung gegen gram-positive und -negative Erreger**

| **MIC [%]** | MRSA | Enterococcus | Streptococcus pneumoniae | Staphylococcus epidermis | E. coli | Klebsiella pneumoniae | *Enterobacter* | Pseudomonas aeroginosa | Clostridium def. | Salmonella |
|---|---|---|---|---|---|---|---|---|---|---|
| **Beispiel 1** | >0,0016 | >0,0002 | >0,0016 | >0,0008 | >0,0016 | >0,025 | >0,003 | >0,003 | >0,0008 | >0,003 |
| **Beispiel 2** | >0,0008 | >0,0008 | >0,0008 | >0,0002 | >0,0008 | >0,025 | >0,0008 | >0,0016 | >0,0004 | >0,0008 |
| **Beispiel 3** | >0,0004 | >0,0008 | >0,0008 | >0,0004 | >0,0008 | n.b. | >0,0016 | >0,003 | >0,0004 | >0,0008 |
| **Beispiel 4** | >0,003 | >0,003 | >0,003 | >0,0016 | >0,0063 | n.b. | >0,0125 | >0,0125 | >0,003 | >0,0125 |
| **Beispiel 5** | >0,0008 | >0,0002 | >0,0004 | >0,0004 | >0,0016 | >0,0125 | >0,0016 | >0,003 | >0,0004 | >0,0016 |
| **Beispiel 6** | >0,025 | >0,0125 | >0,025 | >0,025 | >0,025 | >0,05 | >0,025 | >0,025 | >0,025 | >0,05 |
| **Beispiel 7** | >0,0002 | >0,0002 | >0,0002 | >0,0002 | >0,0008 | >0,0004 | >0,0008 | >0,0008 | >0,0008 | >0,0008 |
| **Beispiel 8** | >0,0004 | >0,0004 | >0,0004 | >0,0004 | >0,0008 | >0,0008 | >0,0008 | >0,0008 | >0,0008 | >0,0008 |
| **Beispiel 9** | >0,0125 | >0,003 | >0,0125 | >0,0125 | >0,0008 | >0,0008 | >0,025 | >0,025 | >0,025 | >0,025 |
| **Beispiel 10** | >0,0125 | >0,0125 | >0,0125 | >0,0125 | >0,025 | >0,025 | >0,025 | >0,025 | >0,025 | >0,025 |
| **Vergleichs-beispiel 1** | >0,001 | >0,008 | >0,004 | >0,001 | >0,016 | >0,02 | >0,008 | >0,02 | n.b. | >0,03 |

**Tabelle 2 - Wirkung gegen Pilze und Hefen**

| **MIC [%]** | Candida albicans | Candida papillosis | Candida glabrata | Candida krusei | Aspergillus terreus | Aspergilus fumigates | Fusarium rosei | Trichophyton sp. | Alternarria sp. | Microsporum canis | Dematiacea sp. |
|---|---|---|---|---|---|---|---|---|---|---|---|
| **Beispiel 1** | >0,025 | >0,025 | >0,025 | >0,025 | >0,05 | >0,05 | >0,05 | >0,025 | >0,025 | >0,025 | >0,025 |
| **Beispiel 2** | >0,025 | >0,025 | >0,025 | >0,025 | >0,05 | >0,05 | >0,025 | >0,025 | >0,05 | >0,025 | >0,05 |
| **Beispiel 3** | >0,025 | >0,025 | >0,025 | >0,025 | >0,05 | >0,05 | >0,025 | >0,025 | >0,05 | >0,025 | >0,05 |
| **Beispiel 4** | >0,05 | >0,05 | >0,05 | >0,05 | >0,1 | >0,1 | >0,1 | >0,05 | >0,05 | >0,05 | >0,1 |
| **Beispiel 5** | >0,025 | >0,025 | >0,025 | >0,025 | >0,05 | >0,05 | >0,025 | >0,025 | >0,05 | >0,025 | >0,05 |
| **Beispiel 6** | >0,05 | >0,05 | >0,05 | >0,05 | >0,05 | >0,05 | >0,05 | | | | |
| **Beispiel 7** | >0,025 | >0,025 | >0,025 | >0,025 | >0,05 | >0,05 | >0,025 | | | | |
| **Beispiel 8** | >0,025 | >0,025 | >0,025 | >0,025 | >0,05 | >0,05 | >0,05 | | | | |
| **Beispiel 9** | >0,05 | >0,05 | >0,05 | >0,05 | >0,05 | >0,05 | >0,05 | | | | |
| **Beispiel 10** | >0,05 | >0,05 | >0,05 | >0,05 | >0,05 | >0,05 | >0,05 | | | | |
| **Vergleichs -beispiel 1** | >0,008 | >0,03 | >0,02 | >0,02 | >0,02 | >0,03 | >0,03 | >0,02 | >0,02 | >0,03 | >0,02 |

**Tabelle 3 - Wirkung gegen Viren**

| **MIC [%]** | Influenza A | Influenza B | Humanes Rhinovirus |
|---|---|---|---|
| **Beispiel 1** | >0,0016 | >0,0016 | >0,0016 |
| **Beispiel 2** | >0,0016 | >0,0016 | >0,0016 |
| **Beispiel 3** | >0,0016 | >0,0016 | >0,0016 |
| **Beispiel 4** | >0,003 | >0,003 | >0,003 |
| **Beispiel 5** | >0,0016 | >0,0016 | >0,0016 |
| **Beispiel 6** | >0,025 | >0,05 | >0,025 |
| **Beispiel 7** | >0,0016 | >0,0016 | >0,0016 |
| **Beispiel 8** | >0,0032 | >0,0016 | >0,0032 |
| **Beispiel 9** | >0,0125 | >0,0125 | >0,0125 |
| **Beispiel 10** | >0,0125 | >0,0125 | >0,0125 |
| **Vergleichsbeispiel 1** | >0,035 | >0,008 | >0,008 |

## Patentansprüche

1. Verfahren zur Herstellung von Polykondensationsprodukten von Guanidin, Aminoguanidin oder Diaminoguanidin G mit einem oder mehreren Benzyl- oder Allyl-Derivaten BA nach dem folgenden Reaktionsschema: 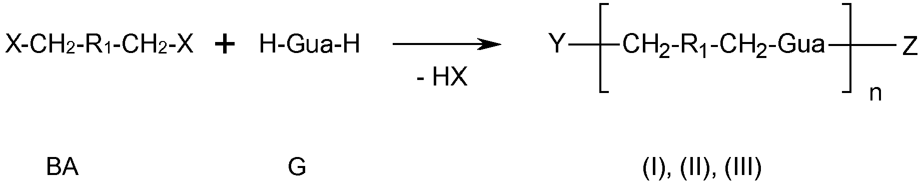 worin
X jeweils unabhängig für eine Abgangsgruppe steht;
R₁ jeweils unabhängig entweder für ein aromatisches Ringsystem mit zumindest einem aromatischen Ring, das gegebenenfalls ein oder mehrere Heteroatome, ausgewählt aus O, N und S enthält und das gegebenenfalls mit einer oder zwei Vinylgruppen substituiert ist, an die die Gruppe(n) -CH₂-X gebunden ist/sind, oder für Ethylen steht; Gua für einen Guanidindiyl-, Aminoguanidindiyl- oder Diaminoguanidindiyl-Rest steht;
Y für H-Gua steht und
Z für H steht; oder
Y und Z zusammen für eine chemische Bindung stehen, um eine zyklische Struktur zu ergeben; und
n ≥ 2 ist;
wobei zumindest ein Benzyl- oder Allyl-Derivat BA einer Polykondensationsreaktion mit einem Überschuss an Guanidin, Aminoguanidin oder Diaminoguanidin G unter Abspaltung von HX unterzogen wird, um ein Polyguanidin der nachstehenden Formel (I), (II) oder (III): 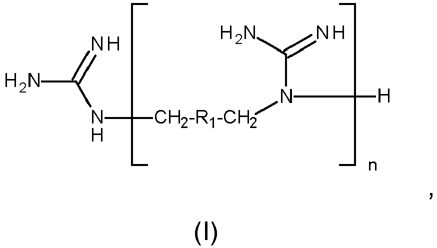 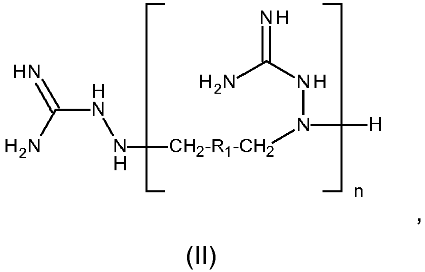 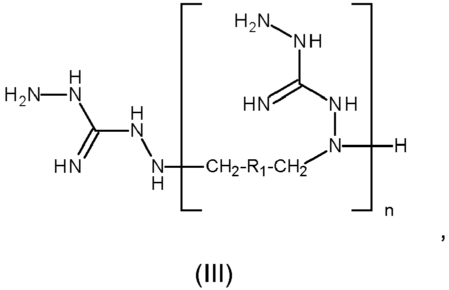 oder mit einer durch Ringschluss unter Eliminierung eines entsprechenden Guanidins erhaltenen zyklischen Struktur, oder ein Salz des Polyguanidins zu ergeben.

2. Verfahren nach Anspruch 1, **dadurch gekennzeichnet, dass** R₁ aus zweiwertigen Resten von gegebenenfalls substituiertem Benzol, Divinylbenzol, Furan, Pyrrol, Thiophen, Pyridin, Biphenyl, Fluoren und Ethylen ausgewählt ist.

3. Verfahren nach Anspruch 2, **dadurch gekennzeichnet, dass** R₁ aus zweiwertigen Resten von Benzol, Divinylbenzol, Pyridin, Biphenyl und Ethylen ausgewählt ist.

4. Verfahren nach einem der Ansprüche 1 bis 3, **dadurch gekennzeichnet, dass** die Abgangsgruppe aus Chlor, Brom, Iod, Mesylat, Triflat und Tosylat ausgewählt ist.

5. Verfahren nach einem der Ansprüche 1 bis 4, **dadurch gekennzeichnet, dass** das zumindest eine zumindest eine Benzyl- oder Allyl-Derivat BA mit Guanidin, Aminoguanidin oder Diaminoguanidin G durch Erhitzen der Reaktanten auf eine Temperatur oberhalb ihrer Schmelztemperaturen umgesetzt wird.

6. Verfahren nach einem der Ansprüche 1 bis 5, **dadurch gekennzeichnet, dass** die Reaktion über einen Zeitraum von zumindest 2 h, vorzugsweise zumindest 3 h, durchgeführt wird.

7. Polyguanidin, das der nachstehenden Formel (I) entspricht: 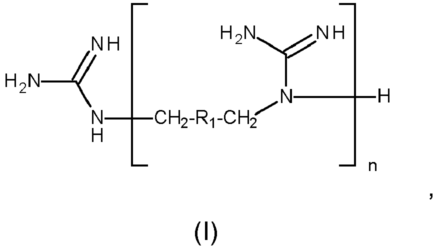 worin R₁ entweder für ein aromatisches Ringsystem mit zumindest einem aromatischen Ring, das gegebenenfalls ein oder mehrere Heteroatome, ausgewählt aus O, N und S enthält und das gegebenenfalls mit einer oder zwei Vinylgruppen substituiert ist, an die die Gruppe(n) -CH₂-X gebunden ist/sind, oder für Ethylen steht und worin n ≥ 2 ist,
oder das eine durch Ringschluss unter Eliminierung eines Guanidins erhaltene zyklische Struktur aufweist.

8. Polyguanidin, das der nachstehenden Formel (II) entspricht: 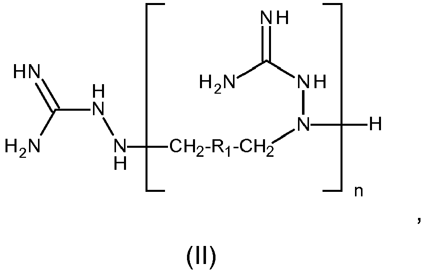 worin R₁ entweder für ein aromatisches Ringsystem mit zumindest einem aromatischen Ring, das gegebenenfalls ein oder mehrere Heteroatome, ausgewählt aus O, N und S enthält und das gegebenenfalls mit einer oder zwei Vinylgruppen substituiert ist, an die die Gruppe(n) -CH₂-X gebunden ist/sind, oder für Ethylen steht und worin n ≥ 2 ist,
oder das eine durch Ringschluss unter Eliminierung eines Aminoguanidins erhaltene zyklische Struktur aufweist.

9. Polyguanidin, das der nachstehenden Formel (III) entspricht: 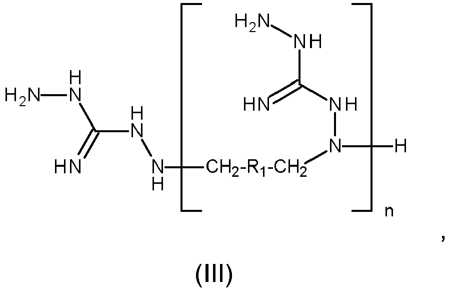 worin R₁ entweder für ein aromatisches Ringsystem mit zumindest einem aromatischen Ring, das gegebenenfalls ein oder mehrere Heteroatome, ausgewählt aus O, N und S enthält und das gegebenenfalls mit einer oder zwei Vinylgruppen substituiert ist, an die die Gruppe(n) -CH₂-X gebunden ist/sind, oder für Ethylen steht und worin n ≥ 2 ist,
oder das eine durch Ringschluss unter Eliminierung eines Diaminoguanidins erhaltene zyklische Struktur aufweist.

10. Polyguanidin nach einem der Ansprüche 7 bis 9, **dadurch gekennzeichnet, dass** R₁ aus zweiwertigen Resten von gegebenenfalls substituiertem Benzol, Divinylbenzol, Furan, Pyrrol, Thiophen, Pyridin, Biphenyl, Fluoren und Ethylen ausgewählt ist.

11. Polyguanidin nach Anspruch 10, **dadurch gekennzeichnet, dass** R₁ aus zweiwertigen Resten von Benzol, Divinylbenzol, Pyridin, Biphenyl und Ethylen ausgewählt ist.

12. Polyguanidin, wie in einem der Ansprüche 7 bis 11 definiert, zur Verwendung als Antiinfektivum.

13. Polyguanidin zur Verwendung nach Anspruch 12, **dadurch gekennzeichnet, dass** das Polyguanidin zur Bekämpfung bakterieller, viraler oder Pilz-Infektionen bei einem menschlichen oder tierischen Patienten, gegebenenfalls durch topische oder systemische Verabreichung und gegebenenfalls in Form eines Arzneimittels oder einer pharmazeutischen Zusammensetzung, dient.

14. Verwendung eines Polyguanidins, wie in einem der Ansprüche 7 bis 11 definiert, als antimikrobielles Mittel *ex vivo,* wobei das Polyguanidin gegebenenfalls als Wirkkomponente von antimikrobiellen Anstrichen, Beschichtungen, Folien oder Membranen dient.

15. Arzneimittel oder pharmazeutische Zusammensetzung zur Bekämpfung bakterieller Infektionen bei einem menschlichen oder tierischen Patienten, das/die ein Polyguanidin nach einem der Ansprüche 7 bis 11 als Antiinfektivum umfasst.

16. Arzneimittel oder pharmazeutische Zusammensetzung nach Anspruch 15, **dadurch gekennzeichnet, dass** es/sie weiters zumindest einen pharmazeutisch annehmbaren Träger oder Exzipienten und gegebenenfalls ein oder mehrere Adjuvantien und/oder einen oder mehrere andere Wirkstoffe umfasst.

17. Arzneimittel oder pharmazeutische Zusammensetzung nach Anspruch 16, **dadurch gekennzeichnet, dass** es/sie zumindest einen anderen Wirkstoff umfasst, der ebenfalls antiinfektiv wirkt, und/oder zumindest einen anderen Wirkstoff umfasst, der gegen ein anderes Leiden als eine bakterielle Infektion wirksam ist.

## Claims

1. A method for preparing polycondensation products from guanidine, aminoguanidine or diaminoguanidine G with one or more benzyl or allyl derivatives BA according to the following reaction scheme: 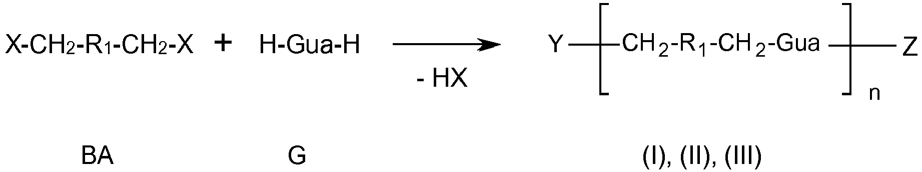 wherein
each X independently represents a leaving group;
each R₁ independently represents either an aromatic ring system with at least one aromatic ring, optionally containing one or more hetero atoms selected from O, N and S and optionally being substituted with one or two vinyl groups to which the -CH₂-X group(s) is/are bound, or represents ethylene;
Gua represents a guanidindiyl, aminoguanidindiyl or diaminoguanidindiyl residue;
Y represents H-Gua, and
Z represents H; or
Y and Z together represent a chemical bond to obtain a cyclic structure; and
n is ≥ 2;
wherein at least one benzyl or allyl derivative BA is subjected to a polycondensation reaction with excess guanidine, aminoguanidine or diaminoguanidine G upon elimination of HX in order to provide a polyguanidine corresponding to the following formula (I), (II) or (III): 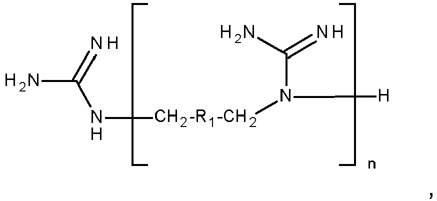 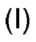 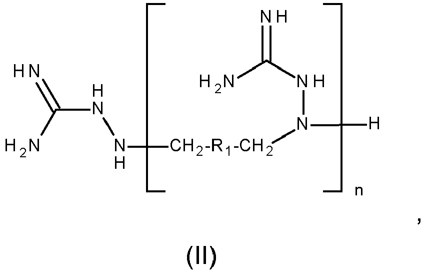 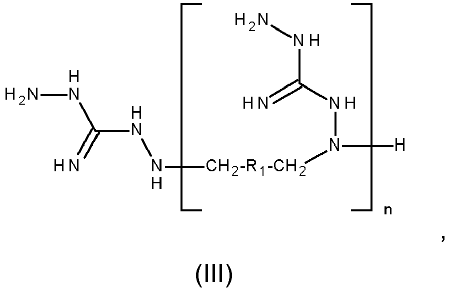 or having a cyclic structure resulting from cyclization upon elimination of a corresponding guanidine, or a salt of said polyguanidine.

2. The method of Claim 1, **characterized in that** R₁ is selected from divalent residues of optionally substituted benzene, divinylbenzene, furan, pyrrole, thiophene, pyridine, biphenyl, fluorene or ethylene.

3. The method of Claim 2, **characterized in that** R₁ is selected from divalent residues of benzene, divinylbenzene, pyridine, biphenyl or ethylene.

4. The method of any one of the Claims 1 to 3, **characterized in that** the leaving group is selected from chlorine, bromine, iodine, mesylate, triflate or tosylate.

5. The method of any one of the Claims 1 to 4, **characterized in that** the at least one benzyl or allyl derivative BA is reacted with guanidine, aminoguanidine or diaminoguanidine G by heating the reactants at a temperature above their melting temperatures.

6. The method of any one of the Claims 1 to 5, **characterized in that** the reaction is conducted for a period of at least 2 h, preferably at least 3 h.

7. A polyguanidine of the following formula (I): 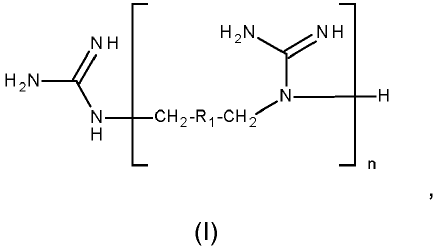 wherein R₁ represents either an aromatic ring system with at least one aromatic ring, optionally containing one or more heteroatoms selected from O, N and S and optionally being substituted with one or two vinyl groups to which the -CH₂-X group(s) is/are bound, or represents ethylene and wherein n is ≥ 2,
or having a cyclic structure resulting from cyclization upon elimination of a guanidine.

8. A polyguanidine of the following formula (II): 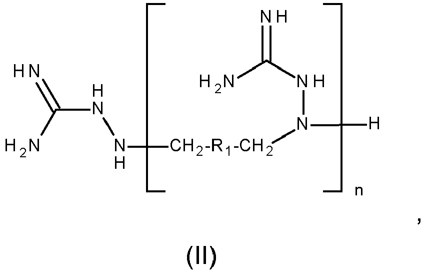 wherein R₁ represents either an aromatic ring system with at least one aromatic ring, optionally containing one or more heteroatoms selected from O, N and S and optionally being substituted with one or two vinyl groups to which the -CH₂-X group(s) is/are bound, or represents ethylene and wherein n is ≥ 2,
or having a cyclic structure resulting from cyclization upon elimination of a aminoguanidine.

9. A polyguanidine of the following formula (III): 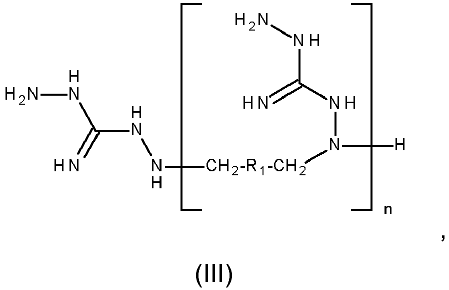 wherein R₁ represents either an aromatic ring system with at least one aromatic ring, optionally containing one or more heteroatoms selected from O, N and S and optionally being substituted with one or two vinyl groups to which the group(s) -CH₂-X is/are bound, or represents ethylene and wherein n is ≥ 2,
or having a cyclic structure resulting from cyclization upon elimination of a diaminoguanidine.

10. The polyguanidine of any one of the Claims 7 to 9, **characterized in that** R₁ is selected from divalent residues of optionally substituted benzene, divinylbenzene, furan, pyrrole, thiophene, pyridine, biphenyl, fluorene or ethylene.

11. The polyguanidine of Claim 10, **characterized in that** R₁ is selected from divalent residues of benzene, divinylbenzene, pyridine, biphenyl or ethylene.

12. The polyguanidine as defined in any one of the Claims 7 to 11 for use as an antiinfective agent.

13. The polyguanidine for the use of Claim 12, **characterized in that** the polyguanidine is for combating bacterial, viral or fungal infections in a human or animal patient, optionally by means of topical or systemic administration and optionally in the form of a drug or a pharmaceutical composition.

14. The use of a polyguanidine as defined in any one of the Claims 7 to 11 as an *ex vivo* antimicrobial agent, said polyguanidine optionally serving as an active component of antimicrobial paints, coatings, foils, or membranes.

15. A drug or pharmaceutical composition for combating bacterial infections in a human or animal patient, comprising a polyguanidine of any one of the Claims 7 to 11 as an antiinfective agent.

16. The drug or pharmaceutical composition of Claim 15, **characterized in that** it further comprises at least one pharmaceutically acceptable carrier or excipient and optionally one or more adjuvants and/or one or more further active agents.

17. The drug or pharmaceutical composition of Claim 16, **characterized in that** it comprises at least one further active agent that also has an antiinfective effect and/or at least one further active agent that is effective against a condition other than bacterial infections.

## Revendications

1. Procédé de préparation de produits de polycondensation de guanidine, d'aminoguanidine ou de diaminoguanidine G avec un ou plusieurs dérivés benzyle ou allyle BA selon le schéma réactionnel suivant: 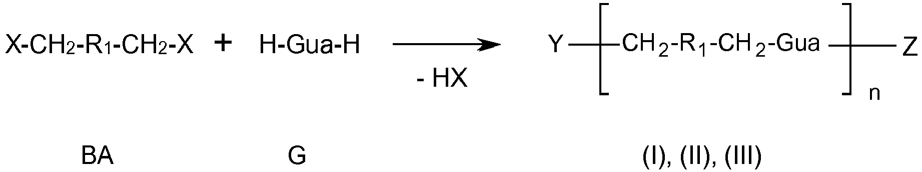 dans lequel
X représente respectivement indépendamment un groupe partant;
chaque R₁ représente séparément soit une combinaison cyclique aromatique comprenant au moins un cycle aromatique, qui contient éventuellement un ou plusieurs hétéroatomes choisis parmi O, N et S et qui présente éventuellement une substitution par un ou deux groupes vinyle, auxquels sont liés le ou les groupes -CH₂-X, soit éthylène; Gua représente un résidu guanidindiyle, aminoguanidindiyle ou diaminoguanidindiyle;
Y représente H-Gua et
Z représente H; ou
Y et Z représentent ensemble une liaison chimique, permettant d'obtenir une structure cyclique; et
n étant ≥ 2 ;
au moins un dérivé benzyle ou allyle BA étant soumis à une réaction de polycondensation avec un excédent de guanidine, d'aminoguanidine ou de diaminoguanidine G, produisant la dissociation de HX, pour donner une polyguanidine selon la formule (I), (II), (III) ci-dessus : 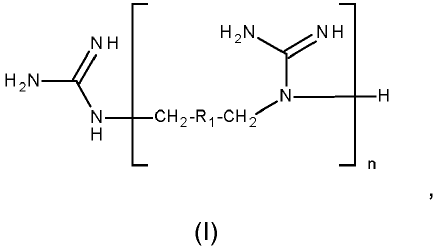 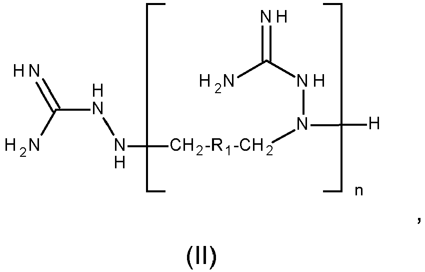 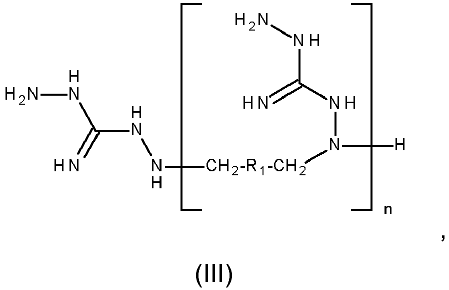 ou qui présente une structure cyclique obtenue par cyclisation après l'élimination d'une guanidine respective, ou un sel de la polyguanidine.

2. Procédé selon la revendication 1, **caractérisé en ce que** R₁ est choisi parmi des résidus bivalents de benzène, divinylbenzène, furanne, pyrrole, thiophène, pyridine, biphényle, fluorène et éthylène éventuellement substitué.

3. Procédé selon la revendication 2, **caractérisé en ce que** R₁ est choisi parmi des résidus bivalents de benzène, divinylbenzène, pyridine, biphényle et éthylène.

4. Procédé selon une quelconque des revendications 1 à 3, **caractérisé en ce que** le groupe partant est choisi parmi chlore, brome, iode, mésylate, triflate ou tosylate.

5. Procédé selon une quelconque des revendications 1 à 4, **caractérisé en ce que** l'au moins un dérivé benzyle ou allyle BA est mis à réagir avec de la guanidine, aminoguanidine ou diaminoguanidine G par réchauffement des réactifs à une température au-delà de sa température de fusion.

6. Procédé selon une quelconque des revendications 1 à 5, **caractérisé en ce que** la réaction est effectué pour une durée d'au moins 2 h, de préférence au moins 3 h.

7. Polyguanidine selon la formule (I) ci-dessus : 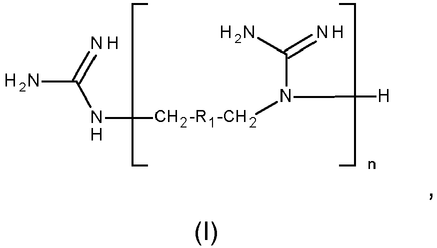 dans laquelle R₁ représente soit une combinaison cyclique aromatique comprenant au moins un cycle aromatique, qui contient éventuellement un ou plusieurs hétéroatomes choisis parmi O, N et S et qui présente éventuellement une substitution par un ou deux groupes vinyle, auxquels sont liés le ou les groupes -CH₂-X, soit éthylène; et n est ≥ 2, ou
ou qui présente une structure cyclique obtenue par cyclisation après l'élimination d'une guanidine.

8. Polyguanidine selon la formule (II) ci-dessus : 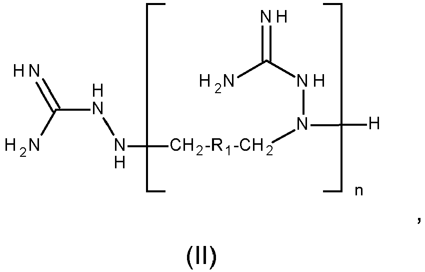 dans laquelle R₁ représente soit une combinaison cyclique aromatique comprenant au moins un cycle aromatique, qui contient éventuellement un ou plusieurs hétéroatomes choisis parmi O, N et S et qui présente éventuellement une substitution par un ou deux groupes vinyle, auxquels sont liés le ou les groupes -CH₂-X, soit éthylène; et n est ≥ 2, ou
ou qui présente une structure cyclique obtenue par cyclisation après l'élimination d'une aminoguanidine.

9. Polyguanidine selon la formule (III) ci-dessus : 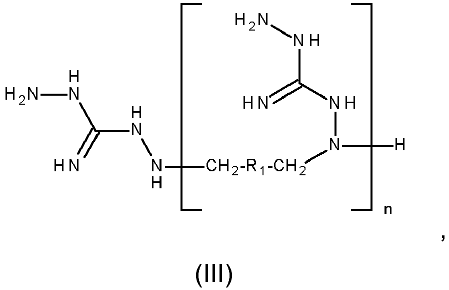 dans laquelle R₁ représente soit une combinaison cyclique aromatique comprenant au moins un cycle aromatique, qui contient éventuellement un ou plusieurs hétéroatomes choisis parmi O, N et S et qui présente éventuellement une substitution par un ou deux groupes vinyle, auxquels sont liés le ou les groupes -CH₂-X, soit éthylène; et n est ≥ 2,
ou qui présente une structure cyclique obtenue par cyclisation après l'élimination d'une diaminoguanidine.

10. Polyguanidine selon une quelconque des revendications 7 à 9, **caractérisée en ce que** R₁ est choisi parmi des résidus bivalents de benzène, divinylbenzène, furanne, pyrrole, thiophène, pyridine, biphényle, fluorène et éthylène éventuellement substitué.

11. Polyguanidine selon la revendication 10, **caractérisée en ce que** R₁ est choisi parmi des résidus bivalents de benzène, divinylbenzène, pyridine, biphényle et éthylène.

12. Polyguanidine selon une quelconque des revendications 7 à 11, pour usage comme anti-infectueux.

13. Polyguanidine pour l'usage selon la revendication 12, **caractérisée en ce que** la polyguanidine est destinée à combattre des infections bactériales, virales ou fongiques chez un individu humain ou animal, éventuellement par administration topique ou systémique et éventuellement sous forme d'un médicament ou d'une composition pharmaceutique.

14. Usage d'une polyguanidine selon la définition d'une quelconque des revendications 7 à 11, comme agent antimicrobien *ex vivo,* la polyguanidine servant éventuellement de composante active pour peintures, revêtements, films ou membranes antimicrobien(ne)s.

15. Médicament ou composition pharmaceutique pour combattre des infections bactériennes chez un individu humain ou animal, comprenant une polyguanidine selon une quelconque des revendications 7 à 11 comme anti-infectueux.

16. Médicament ou composition pharmaceutique selon la revendication 15, **caractérisé en ce que**, de plus, qu'il/elle comprend au moins un porteur ou excipient pharmaceutiquement acceptable et éventuellement un ou plusieurs adjuvants et/ou un ou plusieurs autres substances actives.

17. Médicament ou composition pharmaceutique selon la revendication 16, **caractérisé en ce qu'**il/elle comprend au moins une autre substance active, qui est anti-infectueuse aussi, et/ou au moins une autre substance active, qui est efficace contre un autre mal qu'une infection bactérienne.
